Europäisches Patentamt

European Patent Office

Office européen des brevets

(19)

(11) Veröffentlichungsnummer : **0 111 699**
**B1**

(12) **EUROPÄISCHE PATENTSCHRIFT**

(45) Veröffentlichungstag der Patentschrift :
25.03.87

(51) Int. Cl.⁴ : **C 07 D307/48, C 07 C 51/48,
D 21 C 11/00**

(21) Anmeldenummer : 83110853.5

(22) Anmeldetag : 29.10.83

(54) Verfahren zur Gewinnung von Furfural und Monocarbonsäuren aus wässrigen Lösungen.

(30) Priorität : 17.12.82 AT 4585/82

(43) Veröffentlichungstag der Anmeldung :
27.06.84 Patentblatt 84/26

(45) Bekanntmachung des Hinweises auf die Patenterteilung : 25.03.87 Patentblatt 87/13

(84) Benannte Vertragsstaaten :
BE CH DE FR GB IT LI LU NL SE

(56) Entgegenhaltungen :
EP-A- 0 036 406
EP-A- 0 049 429
DE-A- 1 517 231
GB-A- 1 465 174
US-A- 2 536 732
CHEMICAL ABSTRACTS, Band 88, Nr. 20, 15. Mai
1978, Seite 389, Nr. 142314w, Columbus, Ohio, US
Die Akte enthält technische Angaben, die nach dem
Eingang der Anmeldung eingereicht wurden und die
nicht in dieser Patentschrift enthalten sind.

(73) Patentinhaber : Extraktionstechnik Gesellschaft für
Anlagenbau m.b.H.
Humboldtstrasse 56 Postfach 76 03 69
D-2000 Hamburg 76 (DE)

(72) Erfinder : Marr, Rolf, Prof. Dr.-Ing.
Neue-Welt-Höhe 33
A-8042 Graz (AT)
Erfinder : Siebenhofer, Matthäus, Dr.-Ing.
Evangelimanngasse 19/3/9
A-8010 Graz (AT)
Erfinder : Resch, Michael, Dipl.-Ing.
Grabenstrasse 208
A-8010 Graz (AT)
Erfinder : Bunzenberger, Gerhard, Dipl.-Ing.
Grüne Gasse 38
A-8020 Graz (AT)

(74) Vertreter : Planker, Karl-Josef, Dipl.-Phys.
c/o BABCOCK-BSH AKTIENGESELLSCHAFT Parkstrasse 29 Postfach 4 + 6
D-4150 Krefeld 11 (DE)

## Beschreibung

Die Erfindung betrifft ein Verfahren zur Gewinnung von Furfural und Monocarbonsäuren, insbesondere von Essigsäure, aus wäßrigen Lösungen, insbesondere aus Abwässern der Zellstoffindustrie, durch Flüssig-Flüssigextraktion mit organischen Extraktionsmitteln und anschließende destillative Abtrennung der extrahierten Wertstoffe aus der organischen Phase, wie es im Folgenden näher definiert wird.

In der AT-PS 356 509 ist ein Verfahren zur Rückgewinnung von Chemikalien aus Abwässern von Zellstoffabriken beschrieben, bei dem zur Gewinnung von Furanderivaten und Monocarbonsäuren aus deren verdünnten wäßrigen Lösungen die genannten Verbindungen durch gemeinsame Extraktion mit solvatisierenden Extraktionsmitteln durch einen einstufigen oder mehrstufigen Kontakt mit der wäßrigen Phase in die wasserunlösliche organische Phase übergeführt werden. In einer der Extraktion nachfolgenden Abstreifoperation werden die genannten Verbindungen vom Extraktionsmittel abgetrennt. Aus dieser Rohlösung müssen die Reinkomponenten in mehreren Destillationsverfahren isoliert werden. Diese Vorgehensweise hat jedoch mehrere technologische Nachteile, die die Wirtschaftlichkeit dieses Verfahrens in Frage stellen.

So wird vor allem durch Verwendung von solvatisierenden Lösungsmitteln wie Phosphinoxiden eine beträchtliche Wassermenge, die bis zu 15 Gew.-% betragen kann (bezogen auf die vom Lösungsmittel abgestreifte Säure), mitextrahiert. Ergebnisse dazu wurden von N.L. Ricker, J.N. Michaels und J.C. King in J. Separ. Proc. Technol. 1, 1979, 36-41, sowie J. Wardell und C.J. King in J. Chem. Eng. Data, 23, 1978, 144-148 veröffentlicht.

Weiterhin werden die bei der Extraktion mitgeschleppten Furanderivate in den der Abstreifoperation folgenden Arbeitsschritten mehrfach thermisch hoch belastet, wodurch Verluste durch unkontrollierte Reaktionen dieser Produkte sowie eine wesentliche Beeinträchtigung der Produktqualität der Endprodukte auftreten.

Durch die US-A-2 536 732 ist ein Verfahren zur Herstellung von Furfural bekannt, bei dem das Furfural aus einem wäßrigen Medium mit einem aus der Gruppe der aromatischen Kohlenwasserstoffe, halogenierten aromatischen Kohlenwasserstoffe und chlorierten Diphenyle ausgewählten Extraktionsmittel extrahiert wird, dessen Siedepunkt über dem des Furfurals liegt. Aus der organischen Phase wird das Furfural abdestilliert, und das Extraktionsmittel wird in die Extraktionsstufe zurückgeführt.

Durch die EP-A-49 429 ist ein Verfahren zur Extraktion von organischen Säuren aus wäßriger Lösung bekannt, bei dem die Lösung mit einer Mischung aus einem mit Wasser nicht mischbaren Amin, z. B. Trilaurylamin, einer mit Wasser nicht mischbaren organischen Säure und einem mit Wasser nicht mischbaren organischen Lösungsmittel behandelt wird ; das Lösungsmittel kann z. B. aus aliphatischen Kohlenwasserstoffen bestehen. Dieses Verfahren ist insbesondere zur Extraktion von Zitronensäure, Apfelsäure oder Milchsäure vorgesehen.

Der Erfindung liegt die Aufgabe zugrunde, ein in großtechnischem Maßstab durchführbares Verfahren der eingangs angegebenen Gattung zu schaffen, bei dem unter Vermeidung der oben genannten Nachteile des Standes der Technik das Furfural in einfacher Weise gewonnen wird und bei dem die Verfahrenschemikalien vollständig und problemlos rückführbar sind.

Diese Aufgabe wird gemäß der Erfindung durch die im kennzeichnenden Teil des Patentanspruchs 1 angegebenen Merkmale gelöst.

Zur Lösung der gestellten Aufgabe wird also gemäß der Erfindung vom dem Grundkonzept ausgegangen, Furfural und Monocarbonsäuren getrennt zu extrahieren. In der ersten Extraktionsstufe wird das Furfural mit einem ersten flüssigen Extraktionsmittel mit Lewis-Säuren-Charakter aus der wäßrigen Lösung entfernt. Die erhaltene wäßrige Lösung wird nach Abtrennung des Furfurals in der zweiten Extraktionsstufe mit dem flüssigen zweiten Extraktionsmittel behandelt, um die Monocarbonsäuren zu gewinnen.

Ein großer Vorteil des erfindungsgemäßen Verfahrens liegt darin, daß in der ersten Extraktionsstufe unerwünschte Matrixstoffe, wie Pentosen, Hexosen und Ligninverbindungen, durch Ausflocken problemlos entfernt werden können. Ein besonderer Vorteil besteht darin, daß die Bedingungen eines vollständigen Recycling der Verfahrenschemikalien ohne Probleme zu erfüllen sind.

Bevorzugte Varianten des Verfahrens gemäß der Erfindung sind in den Patentansprüchen 2 bis 13 angegeben sowie im nachstehenden Beschreibungsteil und in den Beispielen ausgeführt.

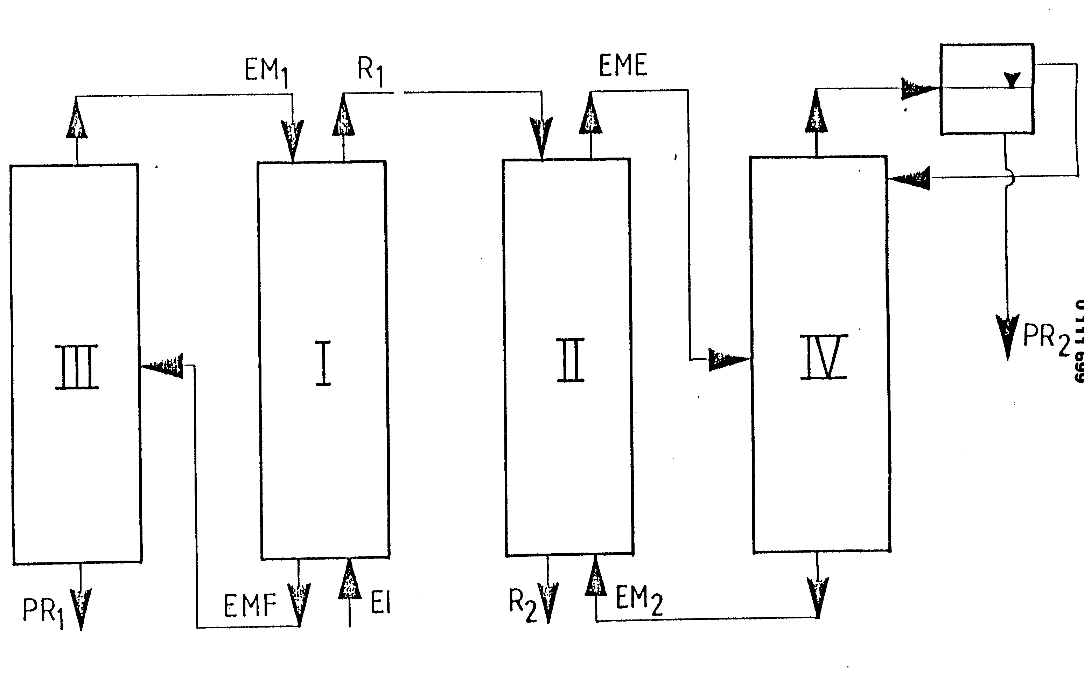

Eine Ausführungsform des erfindungsgemäßen Verfahrens wird anhand von Fig. 1 erläutert, die das Fließschema einer Extraktions/Destillationsanlage für den Betrieb mit Lösungsmitteln zeigt, deren Dichten höher und deren Siedepunkte niedriger als die des Furfurals sind.

Das Rohprodukt (z. B. Brüdenkondensat El) wird im Extraktor I mit dem ersten Extraktionsmittel $EM_1$ in Kontakt gebracht. Dabei wird der Wertstoff (Furfural) in die organische Phase übergeführt. Das Raffinat $R_1$ wird im Extraktor II mit dem zweiten Extraktionsmittel $EM_2$ in Kontakt gebracht. Der Wertstoff (z. B. Essigsäure) wird in die organische Phase EME übergeführt. Beim Extraktionsprozeß für die Gewinnung der Monocarbonsäuren werden gegebenenfalls auch Lösungsmittelreste aus der Furfuralextraktion mitextrahiert. Diese können im anschießenden Destillationsprozeß IV zurückgewonnen werden. Das wertstoffarme Raffinat $R_2$ wird in die biologische Nachreinigung oder in den Vorfluter abgeführt.

Die organische Phase EME wird in der Destillationskolonne IV in das Produkt PR$_2$ (Monocarbonsäure) und das gereinigte zweite Extraktionsmittel EM$_2$ aufgetrennt. Als Kopfprodukt erhält man bei einem Einsatz EI von wäßriger Essigsäure mit weniger als 10 Gew.-% ein herogenes Azeotrop, bestehend aus 1/3 Oberphase (Alkangemisch) und 2/3 Unterphase (bestehend aus mindestens 96 Gew.-% Essigsäure). Die Oberphase wird als externer Rücklauf in die Kolonne zurückgeführt. Das zweite Extraktionsmittel EM$_2$ wird in den Extraktor II zurückgeführt. Die organische Phase EMF wird in der Destillationskolonne III in das Furfural PR$_1$ und in das erste Extraktionsmittel EM$_1$ aufgetrennt. Das erste Extraktionsmittel EM$_1$ wird wieder dem Extraktor I zugeführt.

Die Erfindung wird weiterhin anhand der folgenden Beispiele näher erläutert :

Zur Ermittlung von geeigneten Extraktionsmitteln für Furfural und Monocarbonsäuren bzw. des Einflusses von Modifikatoren bei der Monocarbonsäureextraktion werden folgende Versuche durchgeführt :

Versuch 1 :

100 ml wäßrige Furfurallösung mit einem Furfuralgehalt von 0,120 mol/l wurden bei 18 °C in einem Schütteltrichter mit 100 ml Undecan vermengt. Nach dem Einstellen des Gleichgewichtes wurde der Furfuralgehalt von der wäßrigen Raffinatphase chromatographisch mit 0,091 mol/l bestimmt.

Versuch 2 :

100 ml wäßrige Essigsäure mit einem Gehalt von 0,231 mol/l wurden bei 18 °C in einem Schütteltrichter mit 100 ml Undecan vermengt. Nach dem Einstellen des stationären Zustandes wurde in der wäßrigen Phase ein Essigsäuregehalt von 0,229 mol/l chromatographisch festgestellt.

Versuch 3 :

100 ml wäßriger Lösung mit einem Essigsäuregehalt von 0,756 mol/l wurden bei 18 °C in einem Schütteltrichter mit 450 ml Essigsäureisobutylester vermengt. Nach dem Einstellen des Gleichgewichtes wurden 92 ml wäßriger Phase mit einem Essigsäuregehalt von 0,314 mol/l und 458 ml Extraktphase mit einem Essigsäuregehalt von 0,100 mol/l abgenommen. Der Gehalt an Wasser in der organischen Phase wurde mit 0,971 mol/l bestimmt.

Versuch 4 :

Je 100 ml Essigsäurelösung mit einem Essigsäuregehalt von 0,395 mol/l wurden bei 18 °C in einem Schütteltrichter mit 100 ml Octansäure bzw. 100 ml 1,1,1-Trichloräthan vermengt. Nach dem Einstellen des stationären Zustandes wurde für das System Essigsäure/Octansäure/Wasser ein Essigsäuregehalt im Raffinat von 0,298 mol/l chromatographisch und für das System Essigsäure/1,1,1-Trichloräthan/Wasser ein Raffinatgehalt von 0,382 mol/l titrimetrisch festgestellt.

Versuch 5 :

1 000 ml einer wäßrigen Lösung mit einem Gehalt von 0,102 mol/l Propionsäure wurden bei 18 °C mit 100 ml Extraktionsmittel, bestehend aus 40 Vol. % Tri n-Octyl/n-Decylamin, 40 Vol. % Isodecanol und 20 % Undecan/Decan in einem Schütteltrichter extraktiv behandelt. Nach der Einstellung des stationären Zustandes wurde der Gehalt an Propionsäure in der wäßrigen Phase chromatographisch mit 0,054 mol/l bestimmt.

Versuch 6 :

1 000 ml einer wäßrigen Lösung mit einem Gehalt von 0,211 mol/l Essigsäure wurden bei 18 °C mit 500 ml eines in Versuch 5 beschriebenen Extraktionsmittels in einem Schütteltrichter extraktiv behandelt. Nach der Extraktion wurde der Gehalt an Essigsäure in der wäßrigen Raffinatphase chromatographisch mit 0,072 mol/l bestimmt.

Versuch 7 :

100 ml einer wäßrigen Lösung mit einem Essigsäuregehalt von 0,835 mol/l wurden mit 100 ml Extraktionsmittel, bestehend aus 40 Vol. % Octyldioctylphosphonat und 60 Vol. % Decan/Undecan extrahiert. Nach Einstellung des Gleichgewichtes wurde der Raffinatgehalt an Essigsäure titrimetrisch mit 0,510 mol/l ermittelt. Der Gehalt an Essigsäure in der Extraktphase betrug 0,329 mol/l. Das Volumen der Raffinatphase betrug 98 ml.

Versuch 8 :

3

Es wurden wäßrige Lösungen von Ameisensäure, Essigsäure und Propionsäure hergestellt. Diese Lösungen wurden jeweils einstufig mit einem Lösungsmittel, bestehend aus 30 Vol. % Triisooctylamin, 30 Vol. % Isodecanol und 40 Vol. % Decan/Undecan, extrahiert.

In der folgenden Tabelle sind die titrimetrisch festgestellten Gehalte aufgelistet. Es wurden je 500 ml wäßriger Säurelösung mit 500 ml Extraktionsmittel behandelt.

| Verbindung | Einsatzgehalt mol/l | Raffinatgehalt mol/l | Extraktgehalt mol/l |
|---|---|---|---|
| Ameisens. | 1,150 | 0,267 | 0,865 |
| Essigsäure | 0,879 | 0,350 | 0,520 |
| Propions. | 1,195 | 0,066 | 1,015 |

## Beispiel 1

Eine wäßrige Lösung mit einem Gehalt von 1 Gew.-% Furfural und 8 Gew.-% Essigsäure wurde bei 20 °C in einer kontinuierlichen Gegenstromextraktionskolonne mit 1,1,1-Trichloräthan als erstem Extraktionsmittel bei einem Phasenverhältnis von wäßriger zu organischer Phase von 2,5 extraktiv behandelt. Dabei wurde das Furfural bis auf 20 ppm Restgehalt in der wäßrigen Phase abgetrennt. Die organische Phase wurde in einer Destillationskolonne in das erste Extraktionsmittel und in das Produkt aufgetrennt. Das Produkt hatte einen Furfuralgehalt von 98,4 Gew.-%.

Die wäßrige « Raffinatphase » aus der ersten Extraktionsstufe wurde in einem weiteren Extraktionsschritt mit einem zweiten Extraktionsmittel, bestehend aus 30 Vol. % Triisooctylamin, 30 Vol. % Octyldioctylphosphonat und 40 Vol. % Undecan/Decan, in einer kontinuierlichen Gegenstromextraktionskolonne bei einem Phasenverhältnis von wäßriger Phase zu organischer Phase von 0,8 behandelt. Der Restgehalt an Essigsäure in der wäßrigen Raffinatphase nach der Extraktion betrug 0,4 g/l. Die beladene organische Phase wurde anschließend in einer kontinuierlich arbeitenden Füllkörperkolonne bei 70 mbar destillativ in das Kopfprodukt Essigsäure und das Sumpfprodukt zweites Extraktionsmittel aufgetrennt.

Der Gehalt an Essigsäure im Kopfprodukt war 96,4 Gew.-%.

## Beispiel 2

Eine wäßrige Lösung mit einem Gehalt von 1 Gew.-% Furfural und 2,86 Gew.-% Ameisensäure wurde wie im Beispiel 1 mit 1,1,1-Trichloräthan bei 18 °C extraktiv behandelt. Nach der Furfuralextraktion wurde die wäßrige Raffinatphase mit einem nach Beispiel 1 zusammengesetzten zweiten Extraktionsmittel bei einem Phasenverhältnis von 1,5 in einem kontinuierlich arbeitenden Gegenstromextraktor extraktiv behandelt. Dabei wurde die Ameisensäure bis auf einen Gehalt von 0,15 Gew.-% von der wäßrigen Phase abgetrennt. Der Gehalt an Ameisensäure in der Extraktphase war 6,7 Gew.-%. Bei der destillativen Abtrennung (Arbeitsdruck 95 mbar) wurden pro eingesetztem Liter beladenen Extraktionsmittels 56 ml wäßrige Phase mit einem Gehalt von 84,2 Gew.-% Ameisensäure erhalten.

## Beispiel 3

800 ml einer wäßrigen Lösung mit einem Furfuralgehalt von 0,604 mol/l und einem Essigsäuregehalt von 0,874 mol/l wurde fünfmal mit je 80 ml Chloroform geschüttelt. Nach jedem Extraktionsdurchgang wurde der Furfuralgehalt in der wäßrigen Raffinatphase gaschromatographisch bestimmt. Er betrug 0,195 mol/l nach dem ersten, 0,063 nach dem zweiten, 0,020 nach dem dritten, 0,006 nach dem vierten und 0,002 mol/l nach dem fünften Extraktionsdurchgang. Der gaschromatographisch ermittelte Furfuralgehalt in der Extraktphase war nach dem ersten Extraktionsdurchgang 3,200 mol/l. Nach der Abtrennung des Furfurals betrug das Volumen der wäßrigen Raffinatphase 750 ml.

Aus dieser Lösung wurde die Essigsäure dreimal diskontinuierlich mit einem in Beispiel 1 und 2 beschriebenen Lösungsmittel extrahiert. Das Volumen des Extraktionsmittels war jeweils 500 ml. Nach dem dritten Extraktionsdurchgang wurde der Gehalt an Essigsäure in der wäßrigen Phase titrimetrisch mit 0,19 mol/l festgestellt. Das Volumen der Raffinatphase betrug 710 ml.

### Patentansprüche

1. Verfahren zur Gewinnung von Furfural und Monocarbonsäuren, insbesondere von Essigsäure, aus wäßrigen Lösungen, insbesondere aus Abwässern der Zellstoffindustrie, durch Flüssig-Flüssigextrak-

4

0 111 699

tion mit organischen Extraktionsmitteln und anschließende destillative Abtrennung der extrahierten Wertstoffe aus der organischen Phase,
dadurch gekennzeichnet,
daß die Extraktion in zwei Stufen in der Weise durchgeführt wird,
daß in der ersten Stufe die wäßrige Lösung zur selektiven Abtrennung von Furfural mit einem halogenierten Kohlenwasserstoff behandelt wird, dessen Siedepunkt unter dem des Furfurals liegt,
daß in der zweiten Stufe das Raffinat der ersten Stufe zur Abtrennung der Monocarbonsäuren mit einem mit Wasser nicht mischbaren zweiten Extraktionsmittel mit Lewis-Basen-Charakter behandelt wird,
und daß die aus den organischen Phasen der beiden Stufen wiedergewonnenen Extraktionsmittel je in die entsprechende Stufe zurückgeführt werden.

2. Verfahren nach Anspruch 1, dadurch gekennzeichnet, daß in der ersten Stufe als Extraktionsmittel 1,1,1-Trichloräthan verwendet wird.

3. Verfahren nach Anspruch 1, dadurch gekennzeichnet, daß in der ersten Stufe als Extraktionsmittel Chloroform verwendet wird.

4. Verfahren nach einem der Ansprüche 1 bis 3, dadurch gekennzeichnet, daß das in der zweiten Stufe verwendete Extraktionsmittel ein tertiäres Amin enthält.

5. Verfahren nach Anspruch 4, dadurch gekennzeichnet, daß ein tertiäres Amin mit mindestens 15 C-Atomen im Molekül verwendet wird.

6. Verfahren nach Anspruch 5, dadurch gekennzeichnet, daß ein tertiäres Amin mit 18 bis 30 C-Atomen verwendet wird.

7. Verfahren nach einem der Ansprüche 4 bis 6, dadurch gekennzeichnet, daß das tertiäre Amin mit einem Alkan-Gemisch mit mehr als 6 C-Atomen im Molekül verdünnt ist.

8. Verfahren nach Anspruch 7, dadurch gekennzeichnet, daß die Alkane 10 bis 12 C-Atome pro Molekül enthalten.

9. Verfahren nach einem der Ansprüche 4 bis 8, dadurch gekennzeichnet, daß das in der zweiten Stufe verwendete Extraktionsmittel zusätzlich Modifikatoren enthält, ausgewählt aus der Gruppe Phosphinoxide, Phosphate, Alkanole mit mehr als 6 C-Atomen im Molekül, Ketone und Ester, wie Phosphonsäureester, wobei der Gehalt an Modifikatoren zwischen 5 und 40 % beträgt.

10. Verfahren nach einem der Ansprüche 4 bis 9, gekennzeichnet durch folgende Zusammensetzung des in der zweiten Stufe verwendeten Extraktionsmittels :
5 bis 40 % tertiäres Amin
30 bis 90 % Alkan-Gemisch
5 bis 40 % Modifikatoren

11. Verfahren nach Anspruch 10, gekennzeichnet durch einen Gehalt von 30 % an tertiären Aminen.

12. Verfahren nach Anspruch 10 oder 11, gekennzeichnet durch einen Gehalt von 30 % an Modifikatoren.

13. Verfahren nach Anspruch 10 bis 12, gekennzeichnet durch einen Gehalt von 30 bis 40 % Alkan-Gemisch.

## Claims

1. A method of obtaining furfural and monocarboxylic acids, more particularly acetic acid, from aqueous solutions, more particularly from waste waters from the cellulose industry, by liquid-liquid extraction with organic extraction agents and subsequent separation of the extracted useful substances by distillation from the organic phase,
characterised in
that extraction is performed in two steps as follows :
in the first step, for selective separation of furfural, the aqueous solution is treated with a halogenated hydrocarbon having a boiling-point below that of furfural,
in the second step, for separating the monocarboxylic acids, the raffinate from the first step is treated with a Lewis-base type water-immiscible second extraction agent, and
the extraction agents recovered from the organic phases in the two steps are recycled to the corresponding step.

2. A method according to claim 1, characterised in that the extraction agent used in the first step is 1,1,1-trichloroethane.

3. A method according to claim 1, characterised in that the extraction agent used in the first step is chloroform.

4. A method according to any of claims 1 to 3, characterised in that the extraction agent used in the second step is a tertiary amine.

5. A method according to claim 4, characterised in that a tertiary amine with at least 15 C atoms in the molecule is used.

6. A method according to claim 5, characterised in that a tertiary amine with 18 to 30 C atoms is used.

7. A method according to any of claims 4 to 6, characterised in that the tertiary amine is diluted with an alkane mixture with more than 6 C atoms in the molecule.

5

8. A method according to claim 7, characterised in that the alkanes contain 10 to 12 C atoms per molecule.

9. A method according to any of claims 4 to 8, characterised in that the extraction agent used in the second step also contains modifiers chosen from the following group : phosphine oxides, phosphates, alkanols with more than 6 C atoms in the molecule, ketones and esters such as phosphonic acid esters, the content of modifiers being between 5 and 40 %.

10. A method according to any of claims 4 to 9, characterised in that the extraction agent used in the second step has the following composition :

  5 to 40 % tertiary amine
  30 to 90 % alkane mixture
  5 to 40 % modifiers.

11. A method according to claim 10, characterised by a 30 % content of tertiary amines.

12. A method according to claim 10 or 11, characterised by a 30 % content of modifiers.

13. A method according to claims 10-12, characterised by a 30-40 % content of alkane mixture.


**Revendications**

1. Procédé de récupération de furfural et d'acides monocarboxyliques, en particulier d'acide acétique, à partir de solutions aqueuses, notamment d'eaux résiduaires de l'industrie de la cellulose, par extraction liquide-liquide à l'aide d'agents d'extraction organiques et par isolement ultérieur des substances de valeur extraites par distillation de la phase organique,
caractérisé en ce que
l'extraction est effectuée en deux étapes de manière à traiter,
dans la première étape, la solution aqueuse par un hydrocarbure halogéné dont le point d'ébullition est inférieur à celui du furfural, en vue d'isoler de façon sélective le furfural, à traiter,
dans la deuxième étape, le produit de raffinage de la première étape par un deuxième agent d'extraction non miscible à l'eau présentant un caractère de base de Lewis, pour isoler les acides monocarboxyliques,
et à recycler chaque fois dans l'étape correspondante les agents d'extraction récupérés à partir des phases organiques des deux étapes.

2. Procédé suivant la revendication 1, caractérisé en ce que, dans la première étape, on utilise du 1,1,1-trichloroéthane à titre d'agent d'extraction.

3. Procédé suivant la revendication 1, caractérisé en ce que, dans la première étape, on utilise du chloroforme à titre d'agent d'extraction.

4. Procédé suivant l'une des revendications 1 à 3, caractérisé en ce que l'agent d'extraction utilisé dans la deuxième étape comporte une amine tertiaire.

5. Procédé suivant la revendication 4, caractérisé en ce qu'on utilise une amine tertiaire comprenant au moins 15 atomes de carbone dans la molécule.

6. Procédé suivant la revendication 5, caractérisé en ce qu'on utilise une amine tertiaire comprenant 18 à 30 atomes de carbone.

7. Procédé suivant l'une des revendications 4 à 6, caractérisé en ce que l'amine tertiaire est diluée avec un mélange d'alcanes comportant plus de 6 atomes de carbone dans la molécule.

8. Procédé suivant la revendication 7, caractérisé en ce que les alcanes comportent 10 à 12 atomes de carbone par molécule.

9. Procédé suivant l'une des revendications 4 à 8, caractérisé en ce que l'agent d'extraction utilisé dans la deuxième étape comporte en plus des modificateurs, sélectionnés parmi le groupe des oxydes de phosphine, des phosphates, des alcanols comportant plus de 6 atomes de carbone dans la molécule, des cétones et des esters, tels que des esters d'acide phosphonique, la teneur en modificateurs étant comprise entre 5 et 40 %.

10. Procédé suivant l'une des revendications 4 à 9, caractérisé par la composition suivante de l'agent d'extraction utilisé dans la deuxième étape :

  5 à 40 % d'amine tertiaire
  30 à 90 % de mélange d'alcanes
  5 à 40 % de modificateurs.

11. Procédé suivant la revendication 10, caractérisé par une teneur de 30 % en amines tertiaires.

12. Procédé suivant l'une des revendications 10 et 11, caractérisé par une teneur de 30 % de modificateurs.

13. Procédé suivant l'une des revendications 10 à 12, caractérisé par une teneur de 30 à 40 % de mélange d'alcanes.

0 111 699